# EUROPÄISCHE PATENTSCHRIFT

(11) **EP 1 920 797 B1**
(45) Veröffentlichungstag und Bekanntmachung des Hinweises auf die Patenterteilung: **14.04.2010**
(21) Anmeldenummer: 06090207.9
(22) Anmeldetag: 09.11.2006
(51) Int. Cl.: A61M 39/28, F16K 7/06, A61M 39/22

(54) **Reinigungssystem mit einem Fluidkanal-Wählschalter mit Rollenklemmen**
Cleaning system comprising a fluid channel switch having roller clamps
Système de nettoyage avec selecteur de canal de fluide avec des pinces à rouleau

(43) Veröffentlichungstag der Anmeldung: 14.05.2008
(73) Patentinhaber: BANDELIN electronic GmbH & Co. KG, 12207 Berlin (DE)
(72) Erfinder: Gebauer, Dirk, 12249 Berlin (DE); Hagelstein, Erik, 81249 München (DE); Jung, Rainer, 13125 Berlin (DE); Kähler, Sebastian, 13187 Berlin (DE)
(74) Vertreter: Golkowsky, Stefan

(56) Entgegenhaltungen:
- WO-A1-94/06489
- DE-A1- 1 750 119
- JP-A- 10 160 007
- US-A- 4 282 902
- US-A- 5 117 870
- ANONYMOUS: "Pinch valves and a chemical replenishment system incorporating such pinch valves" RESEARCH DISCLOSURE, MASON PUBLICATIONS, HAMPSHIRE, GB, Bd. 363, Nr. 26, Juli 1994 (1994-07), XP007119850 ISSN: 0374-4353

## Beschreibung

Die vorliegende Erfindung betrifft ein Reinigungssystem nach Anspruch 1.

Beispielsweise in medizinischen Vorrichtungen gibt es oftmals eine Mehrzahl von Kanälen, z.B. innerhalb von Elastomerschläuchen. Je nach Betriebszustand der Vorrichtung werden aber nur einzelne Kanäle genutzt, während die anderen Kanäle zu dieser Zeit verschlossen sind. Dies verlangt üblicherweise einen relativ hohen Regelungsaufwand, außerdem ist die Herstellung der Vorrichtung recht kompliziert, da üblicherweise Ventile zum Öffnen und Verschließen der entsprechenden Kanäle genutzt werden.

Aus dem Stand der Technik sind Kanalwähler zum Öffnen oder Verschließen einer Mehrzahl von eigenreversibel verformbaren Kanälen bekannt. Beispiele dafür sind der US 5 117 870, der US 4 282 902 oder der DE 1 750 119 zu entnehmen. Die WO 94/06489 lehrt die Verwendung eines Ringkanals zum Zusammenführen einer Mehrzahl von Kanälen.

Der vorliegenden Erfindung liegt die Aufgabe zugrunde, ein Reinigungssystem zur Reinigung spülbarer medizinischer Hohlinstrumente mit einer Mehrzahl von fluidleitenden Kanälen zu schaffen, welches einen oder mehrere dieser Kanäle zur Flüssigkeitsleitung offen lässt und die übrigen Kanäle sicher verschließt, wobei hierbei ein enthaltener Kanalwähler durch zu leitende Flüssigkeit nicht kontaminiert werden soll und der Gesamtaufbau einfach und kostengünstig bleiben soll.

Die Aufgabe wird durch ein Reinigungssystem Kanalwähler nach Anspruch 1 gelöst.

Anspruch 1 betrifft ein Reinigungssystem zur Reinigung spülbarer medizinischer Hohlinstrumente mit u.a. einem Kanalwähler für eine Anzahl n von fluidleitenden Kanälen (d.h. zur Leitung von Gasen, pastösen Medien und flüssigen Medien), welche zumindest bereichsweise einen zum Öffnen oder Verschließen des jeweiligen Kanals eigenreversibel verformbaren Kanalabschnitt aufweisen. Der Kanalwähler wiederum weist eine Bewegungseinheit mit Quetschelementen zum gleichzeitigen Quetschen einer Anzahl m von Kanalwandschnitten und damit Öffnen oder Verschließen der zugehörigen m Kanäle auf. Die Anzahl m genügt hierbei der Ungleichung 1 ≤ m ≤ n-1.

Hierdurch wird es möglich, eine Vielzahl von beispielsweise Schläuchen durch den Kanalwähler zu führen und hierbei eine Anzahl von entsprechenden Schläuchen zu quetschen, damit nur ein bzw. mehrere Schläuche ungequetscht sind und damit durch die in dem Inneren dieser Schläuche verlaufenden Kanäle Fluid geleitet werden kann. Hierdurch wird zum einen der Kanalwähler nicht kontaminiert, da das Quetschen der Schläuche bzw. der eigenreversibel verformbaren Kanalabschnitte jeweils von außen erfolgt. Unter "eigenreversibel verformbare Kanalabschnitte" wird ein elastisches bzw. annähernd elastisches Verhalten der Schläuche verstanden, wobei in einem zusammengequetschten Zustand diese Kanalabschnitte bzw. Schläuche keine Flüssigkeit leiten und in einem ungequetschten Zustand sich selber so ausrichten, dass der Strom einer Flüssigkeit/eines Fluids im Inneren wieder ermöglicht ist. In der Erfindung inbegriffen soll selbstverständlich auch die gegenteilige Möglichkeit enthalten sein, dass nämlich der entsprechende Abschnitt von sich heraus geschlossen ist und erst durch entsprechende Quetschung sich öffnet.

Die Anwendung eines solchen Kanalwählers ist insbesondere im Zusammenhang mit dem Dosieren bzw. gezielten Einleiten von Flüssigkeiten interessant. So kann nach Maßgabe des Kanalwählers beispielsweise aus einem einzigen Kanal eine bestimmte Flüssigkeit bzw. ein Fluid gefördert bzw. eingeleitet werden, während die übrigen verschlossen sind. Dies bietet insbesondere in der Getränkeindustrie oder auch in der Medizintechnik weite Anwendungsbereiche.

Vorteilhafte Weiterbildungen werden in den abhängigen Ansprüchen angegeben.

Eine vorteilhafte Weiterbildung sieht vor, dass die Bewegungseinheit trommelförmig oder linear ausgeführt ist. Hierbei ergibt sich durch die kürzeren Ansteuerungszeiten sowie den kompakten Aufbau sicherlich ein Vorteil für die trommelförmige Ausführung, lineare Ausführungen, ovale Ausführungen oder andere Formen sind jedoch nicht ausgeschlossen.

Eine weitere vorteilhafte Weiterbildung sieht vor, dass die Quetschelemente der Bewegungseinheit als vorzugsweise auf Achsen gelagerte Rollen ausgeführt sind. Eine Achslagerung der Rollen ist hierbei nicht zwingend notwenig, wenn beispielsweise die Gleitpaarung mit der Schlauchaußenseite ein ausreichendes Gleiten ohne Beschädigung der beteiligten Partner erlaubt. Üblicherweise ist eine Achslagerung der Rollen jedoch aus Verschleißgründen sinnvoll.

Die Quetschelemente sollten an ihrer den verformbaren Kanalwandabschnitten (also üblicherweise an den Schläuchen) zugewandten Grenzfläche aus einem nicht zu harten Material bestehen, insbesondere Materialien mit einer kleinen Eigenelastizität (beispielsweise Hartgummi oder ein technischer Kunststoff) sind auch zum Toleranzausgleich (beispielsweise wenn verschiedene Temperaturen in unterschiedlichen Kanälen gefahren werden) durchaus sinnvoll.

Vorzugsweise sind die Kanalwandabschnitt als Elastomerschläuche, insbesondere als Silikonschläuche ausgeführt, wie sie im Getränke- oder Medizinbereich oftmals verwendet werden, ausgeführt.

Eine vorteilhafte Ausführungsform sieht außerdem vor, dass die Quetschelemente der Bewegungseinheit bezüglich eines Mittelpunktes und/oder einer Mittelachse des Kanalwählers radial von innen und/oder von außen auf die verformbaren Kanalwandabschnitte quetschen. Hierbei ist von der Einfachheit der Konstruktion und dem Erfordernis kleinbauender Größe her vorteilhaft, dass die Quetschelemente radial von innen auf die verformbaren Kanalwandabschnitte (beispielsweise Schläuche) zugreifen, sind hier jedoch auch Mischformen möglich.

Eine weitere vorteilhafte Weiterbildung sieht vor, dass die Quetschelemente über den Umfang der trommelförmigen Bewegungseinheit im Wesentlichen gleichmäßig verteilt sind. Hierdurch ergeben sich gleichmäßige Ansteuerungszeiten bzw. auch eine entsprechend geringe Baugröße.

Eine besonders vorteilhafte Weiterbildung sieht vor, dass die Bewegungseinheit verschiedene mit Quetschelementen versehene Ebenen aufweist, wobei Quetschelemente der jeweiligen Ebenen vorzugsweise auf gleichem Umfang mit Winkelversatz zueinander gelagert sind.

Zunächst einmal wird durch das Versetzen der entsprechenden Quetschelemente (vorzugsweise Rollen) die Baugröße des Kanalwählers klein gehalten. Hier können Quetschelemente in Form von relativ großen Rollen zum Einsatz kommen. Die Größe der Rollen ist vorteilhaft, da hierdurch ein behutsameres quetschen (stetigeres Quetschen aufgrund kleinerer Steigungen am Außenumfang der Rolle) möglich ist. Nun ist es durch den Versatz der Ebenen möglich, relativ große Rollen zum Einsatz zu bringen, ohne dass diese sich gegenseitig überschneiden. Vorteilhafterweise sind diese Rollen auf gleichem Umfang mit Winkelversatz zueinander versetzt, so dass sich auch eine gleichmäßige Achsbelastung der Bewegungseinheit ergibt.

Beispielsweise wird eine erste Rollenebene mit ersten Rollen und eine zweite Rollenebene mit zweiten Rollen vorgesehen, wobei die Rollenebenen um einen bestimmten Winkel zueinander verdreht sind, die Achspositionen von erster und zweiter Rollenebene stehen hierbei in fester Zuordnung zueinander.

Eine weitere vorteilhafte Weiterbildung sieht vor, dass der Kanalwähler ein Außengehäuse aufweist. Die Bewegungseinheit ist vorzugsweise im Gehäuse des Kanalwählers untergebracht, so dass sich hierdurch eine platzsparende und saubere bzw. schützende Anordnung der Bewegungseinheit ergibt. Vorzugsweise ist die Bewegungseinheit auf einer im Gehäuse des Kanalwählers gelagerten Achse befestigt. Die Bewegungseinheit ist somit bezüglich des Gehäuses motorisch und/oder manuell drehbar. Hierdurch können die entsprechenden Kanalwandabschnitte bzw. Schläuche fest im Gehäuse untergebracht sein, lediglich die Bewegungseinheit dreht sich. Hierdurch ist ein unbeabsichtigtes Verknoten/Verheddern der Schläuche, wie dies bei aufwendigen Ventilanordnungen oftmals der Fall ist, ausgeschlossen.

Das Gehäuse weist vorteilhafterweise Einführöffnungen für Elastomerschläuche, deren Inneres die Kanäle darstellen, auf. Hierdurch wird ein leichtes Einführen der Schläuche ermöglicht, das Einführen kann hierbei in einen "ungequetschten" Kanal vorgenommen werden und die Bewegungseinheit dann (bei der trommelförmigen Variante) beispielsweise weitergedreht werden, um einen weiteren Schlauch in die dann freie Öffnung einführen zu können.

Eine weitere vorteilhafte Weiterbildung sieht vor, dass die Anzahl m der Gleichung m = n-1 genügt. Dies heißt, dass gleichzeitig m Kanäle gequetscht werden, nur ein einziger Kanal verbleibt offen. Hierdurch kann selektiv jeder einzelne Kanal auf Durchfluss bzw. Druckverhältnisse etc. geprüft werden.

Erfindungsgemäß ist vorgesehen, dass der Kanalwähler mindestens eine Pumpe aufweist, welche an bis zu n (vorteilhaft alle n) Kanäle angeschlossen ist zur Erzeugung von Unter- oder Überdruck auf das jeweilige Kanalinnere. Hierdurch wird es möglich, mit einer einzigen Pumpe wahlweise Unterdruck bzw. Überdruck auf das Innere eines einzelnen Kanals auszuüben, ohne dass hierdurch eine Vielzahl von Pumpen notwendig ist. Dies verringert den Steuerungsaufwand und den konstruktiven Aufwand sowie die Kosten des Kanalwählers erheblich.

Weiterhin sieht die Erfindung vor, dass der Kanalwähler einen Ringkanal zum Zusammenführen der n Kanäle aufweist. An diesen Ringkanal kann beispielsweise die oben genannte Pumpe oder auch ein Filtersystem etc. angebaut sein, insbesondere bei der Ausführungsform, bei der lediglich ein einziger Kanal jeweils offen ist wird dieser Ringkanal dann zum Abfluss der jeweiligen Flüssigkeit genutzt. Es gibt kein Vermischen zwischen den einzelnen Kanälen und der Restflüssigkeit, welche eventuell aus einem vorher geöffneten Kanal noch stammt, ist zum einen vernachlässigbar gering bzw. wird sofort weggespült mit der Öffnung des nächsten Kanals.

Die vorliegende Erfindung ist insbesondere zur selektiven Reinigung von Kanälen und/oder in einer Einrichtung zum Mischen bzw. Dosieren von Flüssigkeiten, insbesondere Farben, geeignet.

Weitere vorteilhafte Weiterbildungen werden in den übrigen abhängigen Ansprüchen beschrieben.

Die Erfindung wird nun anhand mehrerer Figuren erläutert. Es zeigen.
- Fig. 1: eine schematische Darstellung eines in ein Reinigungssystem eingebauten Kanalwählers,
- Fig. 2: eine Draufsicht sowie ein Querschnitt eines erfindungsgemäßen Kanalwählers mit zwölf Kanälen,
- Fig. 3: eine Explosionszeichnung des Kanalwählers gemäß Fig. 2, und
- Fig. 4: einen Querschnitt sowie eine Draufsicht eines zum Anbau an einen Kanalwähler gemäß Fig. 2 bzw. Fig. 3 geeignete Anbauplatte mit Ringkanal.

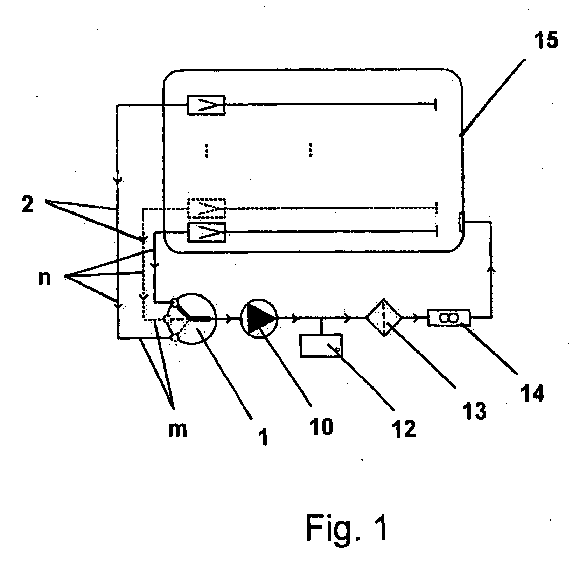

Fig. 1 zeigt ein Schema eines erfindungsgemäßen Reinigungssystems, welches zur Reinigung spülbarer medizinischer Hohlinstrumente geeignet ist. Diese Anwendung ist lediglich ein Beispiel und soll nur der Verdeutlichung dienen. Ein Wasserbad 15 enthält diese Instrumente, welche über Adapter einzeln an Kanäle 2 angeschlossen werden. Die Anzahl dieser Kanäle beträgt n. Sämtliche Kanäle enden in einem Kanalwähler 1. Von den insgesamt n Kanälen werden durch diesen Kanalwähler m Kanäle verschlossen und lediglich ein verbleibender Kanal (beim Kanalwähler 1 durch den fettgedruckten Winkel dargestellt) ermöglicht die Durchleitung von Flüssigkeit aus dem Wasserbad 15 durch den Kanalwähler. An den Kanalwähler angeschlossen ist eine Saugpumpe 10, welche Unterdruck auf die insgesamt n Kanäle bzw. auf den verbleibenden jeweils offenen Kanal ausübt. Dieser Pumpe 10 ist ein Druckschalter 12 nachgeordnet sowie ein Filter 13. Schließlich ist ein Durchflusssensor 14 angeordnet, welcher den Durchfluss durch den gerade geschalteten Kanal bestimmen kann. Nach Passieren des Durchflusssensors 14 erfolgt eine Rückführung der Flüssigkeit in das Wasserbad 15.

Im Folgenden soll insbesondere auf den Kanalwähler 1 und dessen Details eingegangen werden.

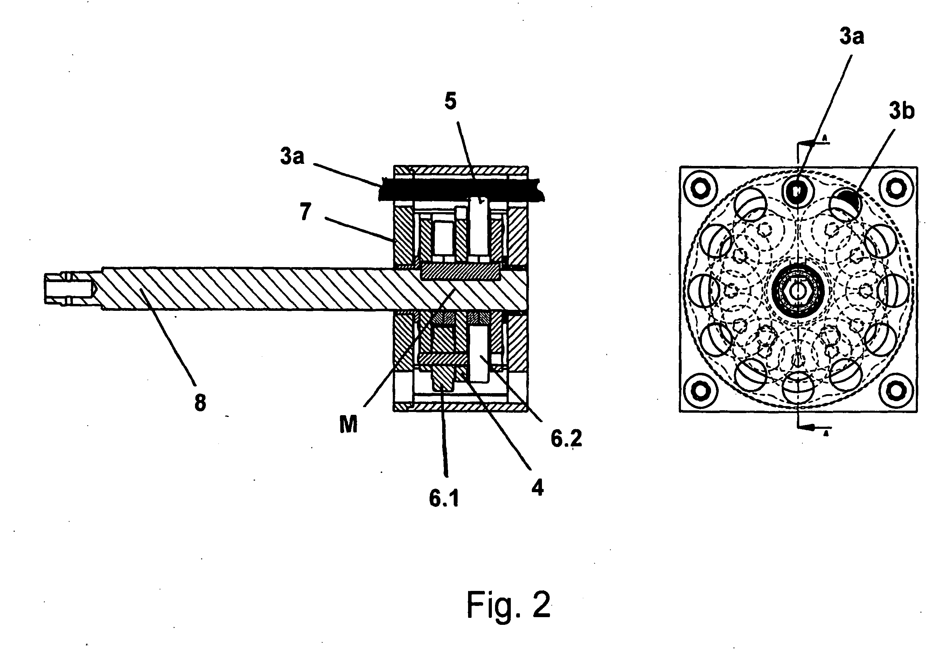

Fig. 2 zeigt einen Kanalwähler 1 für eine Anzahl n von fluidleitenden Kanälen 2, welche jeweils zumindest bereichsweise einen zum Verschließen des jeweiligen Kanals eigen reversibel verformbaren Kanalabschnitt (in der rechtsseitigen Draufsicht mit 3a bzw. 3b bezeichnet) aufweisen.

Der Kanalwähler 1 weist eine Bewegungseinheit 4 mit Quetschelementen 5 in Form von Rollen auf. Diese Quetschelemente 5 dienen dem gleichzeitigen Quetschen einer Anzahl m von Kanalwandabschnitten (in Fig. 2b rechtsseitig am Beispiel von 3b gekennzeichnet) und damit dem Öffnen bzw. Verschließen (vorliegend dem Verschließen) der zugehörigen m Kanäle. Hierbei genügt die Anzahl m der Ungleichung 1 ≤ m ≤ n-1.

Die Bewegungseinheit 4 ist trommelförmig ausgeführt und mittels einer Nut-Feder-Verbindung auf einer Achse 8 angebracht. Diese Achse 8 ist über ein Gleitlager in einem Gehäuse 7 des Kanalwählers gelagert. In Fig. 2 linksseitig ist ein eigenreversibel verformbarer Kanalabschnitt in Form eines Silikonschlauchs gezeigt, wobei der hier in Fig. 2 links gezeigte Schlauch in dem Kanalwähler nicht zusammengepresst ist, so dass er das Durchleiten von Flüssigkeit ermöglicht. Im vorliegenden Beispiel sind sämtliche anderen Kanäle gequetscht, so dass hier durch entsprechende Schläuche keine Flüssigkeit durchleitbar ist. Dies ist beispielsweise in Fig. 2 rechtsseitig zu erkennen, hier sind (abgesehen von dem obersten, mittleren Loch) sämtliche Löcher zum Durchführen von Schläuchen bzw. eigenreversibel verformbaren Kanalabschnitten als gequetscht anzusehen, weil entsprechende Quetschelemente in Form von Rollen 5 einen Druck ausüben würden, so dass sich der in dem mit dem Bezugszeichen 3b gezeigten Zustand einstellen würde.

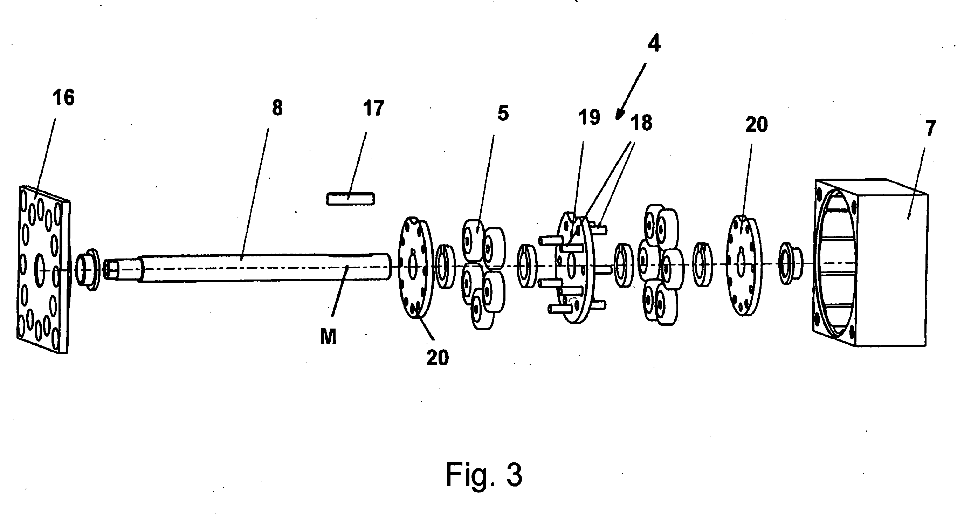

Auf konstruktive Details des Kanalwählers wird jetzt insbesondere mit Bezug auf die Explosionszeichnung in Fig. 3 eingegangen.

Fig. 3 zeigt ein geöffnetes Gehäuse 7, die entsprechende, zu dem Gehäuse gehörende Schließplatte 16 ist linksseitig gezeigt. Die Achse 8, auf welcher die Bewegungseinheit 4 mittels einer Nut-Feder-Verbindung (siehe Feder als Teil 17) angeordnet ist, ist in der Mitte (siehe Mittellinie durch den Mittelpunkt M des Gehäuses 7, gekennzeichnet durch die strichlierte Linie) geführt.

In Fig. 3 ist gut zu erkennen, dass die Quetschelemente 5 der Bewegungseinheit 4 als auf Achsen 18 gelagerte Rollen ausgeführt sind. Diese Achsen stehen nach links und nach rechts (siehe Fig. 3) von einer Zentralplatte 19 ab. Auf der der Zentralplatte abweisenden Seite der Achsen sind hierbei jeweils Komplementärscheiben 20 gezeigt. Insbesondere ist zu sehen, wie sowohl in den Komplementärscheiben 20 als auch in der Zentralplatte 19, jeweils oben eine kleine Ausbuchtung gezeigt ist, welche das ungequetschte Durchführen eines quetschbaren Kanalabschnitts bzw. eines Silikonschlauches erlaubt, in diesem Bereich ist auch keine Rolle gezeigt, welche den Schlauch entsprechend quetschen würde.

Sämtliche der verbleibenden Öffnungen zum Durchführen von Schläuchen (siehe beispielsweise Fig. 2 rechtsseitig) haben einen verringerten Querschnitt, so dass entsprechende Schläuche hier gequetscht würden.

Sämtliche der hier gezeigten Rollen weisen einen Au-ßenring aus Hartgummi auf, welcher entsprechende Silikonschläuche (siehe beispielsweise Kanalabschnitt 3a in Fig. 2) quetscht.

Als eigenreversibel verformbare Kanalabschnitte kommen insbesondere Elastomerschläuche bzw. vorliegend Silikonschläuche in Betracht.

Der Aufbau in Fig. 3 zeigt Rollen, welche jeweils radial "nach außen" drücken, d.h., dass alle Quetschelemente (Rollen) 5 der Bewegungseinheit 4 bezüglich eines Mittelpunkts M des Kanalwählers radial von innen auf die verformbaren Kanalwandabschnitte quetschen.

Der Vorteil dieser Variante ist, dass eine relativ kleine Bauform erreicht werden kann, da sämtliche Rollen im Zentrum des Gehäuses bzw. der Bewegungseinheit angebracht sind.

Außerdem ist zu sehen, dass die Quetschelemente 5 (also die Rollen) über den Umfang der Bewegungseinheit bzw. der Zentralplatte verteilt sind.

Hierbei ist besonders bemerkenswert, dass die Rollen in mehreren Ebenen, vorzugsweise zwei Ebenen, angebracht sind. Linksseitig ist eine erste Ebene (siehe 6.1 in Fig. 2) gezeigt, diese wird von Rollen gebildet, welche auf den linksseitig in Fig. 3 aus der Zentralplatte 19 ragenden Achse bzw. Achsbolzen aufgesteckt werden. Mit diesen Rollen werden fünf Kanäle bzw. Kanalabschnitte gequetscht.

Winkelmäßig hierzu verdreht, aber in fester Zuordnung auf der Zentralplatte 19 sind in Fig. 3 rechtsseitig weitere sechs Rollen gezeigt (s. 6.2 in Fig. 2), welche ebenfalls auf entsprechende Achsbolzen 18 aufgesteckt sind, welche jedoch rechtsseitig aus der Platte 19 herausragen.

Somit werden mit diesen Rollen insgesamt in den jeweiligen Raststufen elf Kanäle gequetscht, so dass der verbleibende zwölfte Kanal jeweils frei ist. Hierbei ist eine Rastung in den jeweiligen "Offenstellungen" der Kanäle gegeben.

Der Vorteil der versetzten Ebenen (es sind auch mehr als zwei Ebenen möglich) ist, dass hier relativ kleinbauend (vor allem in radialer Richtung/Ausdehnung kleinbauend) gebaut werden kann. Trotzdem ist die Rollengröße hier nicht beschränkt, da innerhalb der jeweiligen Ebene nur wenige Rollen gegeben sind, welche somit einen relativ großen Durchmesser haben können ohne sich gegenseitig zu berühren.

Die Achse 8, auf welcher die Bewegungseinheit 4 und mit dieser die entsprechende Teile 19 bzw. 20 und 18 bzw. 5 bewegbar sind, ist motorisch (beispielsweise mit einem Schrittmotor) und/oder manuell drehbar.

Das Gehäuse 7 hat die oben angesprochenen zwölf um die Achse 8 im Wesentlichen gleichmäßigen Öffnungen, in welchen Elastomerschläuche (klemmbare Kanalabschnitte) einführbar sind.

Die vorliegende Ausführungsform zeigt, dass von den insgesamt n Kanälen m = n-1 Kanäle gequetscht werden und lediglich ein einziger Kanal offen ist. Selbstverständlich ist es hier auch möglich, konstruktiv vorzusehen, dass eine beliebige Untermenge gequetscht wird, wobei hier jeweils durch Einrichtung bestimmter Rollen/Quetschelemente eine vorgegebene Geometrie stets beibehalten wird.

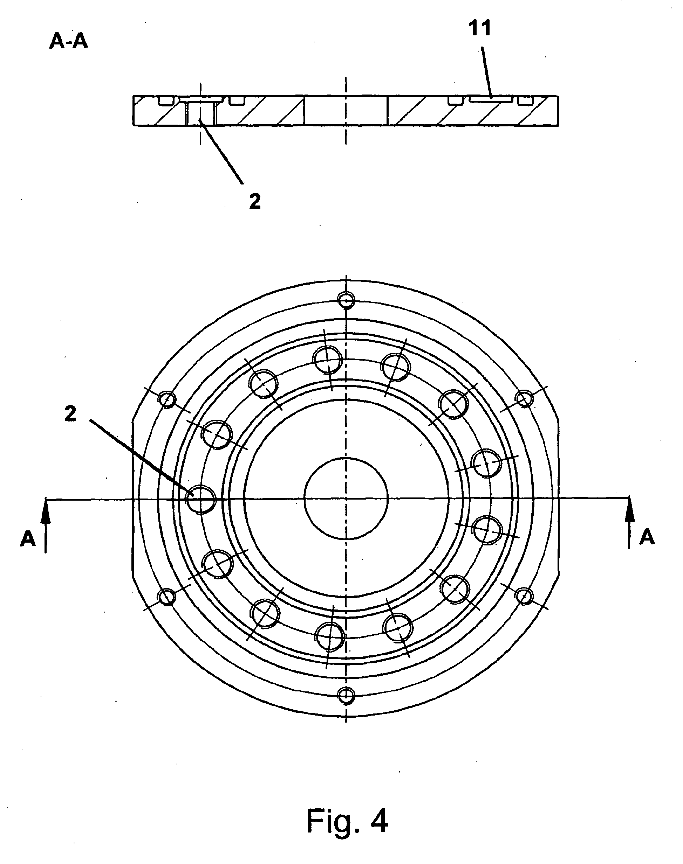

An die in dem Kanalwähler 7 angebrachte Schläuche/Kanalabschnitte kann eine Ringplatte gemäß Fig. 4 angeschraubt werden. Diese zeigt Enden von Kanälen 2, welche in einen Ringkanal 11 münden. Dieser Ringkanal 11 ist wiederum flüssigkeits- bzw. druckdicht mit der Pumpe 11 verbindbar, so dass auf diese Weise wahlweise jeder Kanal mit der vollen Kraft der Pumpe belegt werden kann, ohne dass irgendwelche zusätzlichen Ventile geschaltet werden müssen oder ein Umstecken von Kanälen erfolgen muss, auch ist eine Mehrzahl von Pumpen nicht notwendig.

Die vorliegende Beschreibung ist lediglich beispielhaft zu verstehen. Der Kanalwähler ist auch in anderen Bereichen einsetzbar, beispielsweise zum Mischen und Dosieren von Flüssigkeiten, insbesondere Farben.

Im Unterschied zu Schlauchpumpen, bei welchen eine Förderung durch Quetschung des Schlauches in Schlauchrichtung erfolgt, ist vorzugsweise bei der vorliegenden Erfindung eine Quetschung senkrecht zur Verlaufsrichtung des Schlauches günstig.

## Patentansprüche

1. Reinigungssystem zur Reinigung spülbarer medizinischer Hohlinstrumente, enthaltend einen Behälter für ein Flüssigkeitsbad zum Einlegen der Instrumente,
n Kanäle (2),
einen Kanalwähler (1) für die n Kanäle sowie eine Pumpe (10), wobei
die Instrumente einzeln an die n Kanäle anschließbar und die n Kanäle mit dem Kanalwähler verbunden sind und
die Pumpe zum Ausüben von Unter-oder Übertruck auf die n Kanäle ausgeführt ist, wobei
die n Kanäle jeweils zumindest bereichsweise einen zum Öffnen oder Verschließen des jeweiligen Kanals eigenreversibel verformbaren Kanalabschnitt (3a, 3b) aufweisen, wobei
der Kanalwähler eine Bewegungseinheit (4) mit Quetschelementen (5) zum gleichzeitigen Quetschen einer Anzahl m von Kanalwandabschnitten (3b) und damit Öffnen oder Verschließen der zugehörigen m Kanäle aufweist, wobei m der Ungleichung 1 ≤ m ≤ n-1 genügt, wobei
die Pumpe (10) zur Erzeugung von Unter- oder Überdruck auf das Kanalinnere der bis zu n Kanäle an den Kanalwähler angeschlossen ist und der Kanalwähler einen Ringkanal zum Zusammenführen der n Kanäle aufweist.

2. Reinigungssystem nach Anspruch 1, **dadurch gekennzeichnet, dass** die Bewegungseinheit (4) trommelförmig oder linear ausgeführt ist.

3. Reinigungssystem nach einem der vorhergehenden Ansprüche, **dadurch gekennzeichnet, dass** die Quetschelemente (5) der Bewegungseinheit (4) als vorzugsweise auf Achsen gelagerte Rollen ausgeführt sind.

4. Reinigungssystem nach einem der vorhergehenden Ansprüche, **dadurch gekennzeichnet, dass** die Quetschelemente (5) an ihrer den verformbaren Kanalwandabschnitten zugewandten Grenzfläche aus Hartgummi oder einem technischen Kunststoff bestehen.

5. Reinigungssystem nach einem der vorhergehenden Ansprüche, **dadurch gekennzeichnet, dass** die Kanalwandabschnitte (3a, 3b) als Elastomerschläuche, insbesondere als Silikonschläuche ausgeführt sind.

6. Reinigungssystem nach einem der vorhergehenden Ansprüche, **dadurch gekennzeichnet, dass** die Quetschelemente (5) der Bewegungseinheit (4) bezüglich eines Mittelpunktes (M) und/oder einer Mittelachse des Kanalwählers radial von innen und/oder von außen auf die verformbaren Kanalwandabschnitte (3a, 3b) quetschen.

7. Reinigungssystem nach einem der Ansprüche 2 bis 6, **dadurch gekennzeichnet, dass** die Quetschelemente (5) über den Umfang der trommelförmigen Bewegungseinheit gleichmäßig verteilt sind.

8. Reinigungssystem nach einem der vorhergehenden Ansprüche, **dadurch gekennzeichnet, dass** die Bewegungseinheit (4) verschiedene mit Quetschelementen (5) versehene Ebenen (6.1, 6.2) aufweist, wobei Quetschelemente der jeweiligen Ebenen vorzugsweise umfangsmäßig gegeneinander versetzt gelagert sind.

9. Reinigungssystem nach einem der vorhergehenden Ansprüche, **dadurch gekennzeichnet, dass** der Kanalwähler ein Gehäuse (7) aufweist.

10. Reinigungssystem nach Anspruch 9, **dadurch gekennzeichnet, dass** die Bewegungseinheit (4) im Gehäuse (7) des Kanalwählers untergebracht ist.

11. Reinigungssystem nach Anspruch 9, **dadurch gekennzeichnet, dass** die Bewegungseinheit (4) auf einer im Gehäuse (7) des Kanalwählers gelagerten Achse (8) befestigt ist.

12. Reinigungssystem nach Anspruch 11, **dadurch gekennzeichnet, dass** die Bewegungseinheit bezüglich dem Gehäuse (7) motorisch, pneumatisch, hydraulisch und/oder manuell drehbar ist.

13. Reinigungssystem nach einem der Ansprüche 9 bis 12, **dadurch gekennzeichnet, dass** das Gehäuse (7) des Kanalwählers Einführöffnungen (8) für Elastomerschläuche, deren Inneres Kanäle (2) darstellen, hat.

14. Reinigungssystem nach einem der vorhergehenden Ansprüche, **dadurch gekennzeichnet, dass** die Anzahl m der Gleichung m = n-1 genügt.

## Claims

1. A cleaning system for cleaning flushable medical hollow instruments, containing a vessel for a liquid bath in which to place the instruments, n channels (2),
a channel selector (1) for the n channels and a pump (10),
the instruments being able to be connected individually to the n channels and the n channels being connected to the channel selector, and
the pump being designed to exert an underpressure or overpressure on the n channels, the n channels in each case having, at least in regions, a channel section (3a, 3b) which is deformable in inherently reversible manner for opening or closing the respective channel,
the channel selector having a movement unit (4) with squeezing elements (5) for the simultaneous squeezing of a number m of channel wall sections (3b) and hence the opening or closing of the associated m channels, m satisfying the inequation
1 ≤ m ≤ n-1,
the pump (10) for generating an underpressure or overpressure on the interior of the up to m channels being connected to the channel selector, and the channel selector having a ring channel for bringing together the n channels.

2. A cleaning system according to Claim 1, **characterised in that** the movement unit (4) is designed to be drum-shaped or linear.

3. A cleaning system according to one of the preceding claims, **characterised in that** the squeezing elements (5) of the movement unit (4) are designed as rollers preferably mounted on spindles.

4. A cleaning system according to one of the preceding claims, **characterised in that** the squeezing elements (5) on their boundary surface facing the deformable channel wall sections are made of hard rubber or an engineering plastics material.

5. A cleaning system according to one of the preceding claims, **characterised in that** the channel wall sections (3a, 3b) are in the form of flexible elastomer tubes, in particular as silicone tubes.

6. A cleaning system according to one of the preceding claims, **characterised in that** the squeezing elements (5) of the movement unit (4) squeeze the deformable channel wall sections (3a, 3b) relative to a centre point (M) and/or a centre line of the channel selector radially from inside and/or from outside.

7. A cleaning system according to one of Claims 2 to 6, **characterised in that** the squeezing elements (5) are uniformly distributed over the periphery of the drum-shaped movement unit.

8. A cleaning system according to one of the preceding claims, **characterised in that** the movement unit (4) has different planes (6.1, 6.2) provided with squeezing elements (5), with squeezing elements of the respective planes preferably being mounted offset relative to one another on the periphery.

9. A cleaning system according to one of the preceding claims, **characterised in that** the channel selector has a housing (7).

10. A cleaning system according to Claim 9, **characterised in that** the movement unit (4) is accommodated in the housing (7) of the channel selector.

11. A cleaning system according to Claim 9, **characterised in that** the movement unit (4) is fastened to a spindle (8) mounted in the housing (7) of the channel selector.

12. A cleaning system according to Claim 11, **characterised in that** the movement unit can be rotated relative to the housing (7) by a motor, pneumatically, hydraulically and/or manually.

13. A cleaning system according to one of Claims 9 to 12, **characterised in that** the housing (7) of the channel selector has introduction openings (8) for flexible elastomer tubes, the interiors of which represent channels (2).

14. A cleaning system according to one of the preceding claims, **characterised in that** the number m satisfies the equation m = n - 1.

## Revendications

1. Système de nettoyage pour le nettoyage d'instruments creux médicaux lavables, comportant un récipient pour un bain de liquide destiné à recevoir les instruments,
n canaux (2),
un sélecteur de canaux (1) pour les n canaux et une pompe (10), sachant que
les instruments peuvent être raccordés individuellement aux n canaux, et les n canaux sont reliés au sélecteur de canaux, et
la pompe est conçue pour appliquer une dépression ou surpression dans les n canaux, sachant que
les n canaux comportent chacun au moins dans certaines zones un tronçon de canal (3a, 3b) déformable de manière auto-réversible pour ouvrir ou fermer le canal respectif, sachant que
le sélecteur de canaux comporte une unité de mouvement (4) avec des éléments de pinçage (5) pour pincer simultanément un nombre m de tronçons de parois canaux (3b) et, de ce fait, pour ouvrir ou fermer les m canaux associés, m satisfaisant à l'inéquation 1 ≤ m ≤ n-1, sachant que
la pompe (10) est raccordée au sélecteur de canaux pour générer une dépression ou surpression à l'intérieur des jusqu'à n canaux, et le sélecteur de canaux comporte un conduit annulaire pour la jonction des n canaux.

2. Système de nettoyage selon la revendication 1, **caractérisé en ce que** l'unité de mouvement (4) est réalisée en forme de tambour ou avec une forme linéaire.

3. Système de nettoyage selon l'une quelconque des revendications précédentes, **caractérisé en ce que** les éléments de pinçage (5) de l'unité de mouvement (4) sont réalisés sous la forme de rouleaux, montés de préférence sur des axes.

4. Système de nettoyage selon l'une quelconque des revendications précédentes, **caractérisé en ce que** les éléments de pinçage (5), sont au niveau de leur surface orientée vers les tronçons parois de canaux déformables, réalisés en caoutchouc dur ou dans une matière synthétique technique.

5. Système de nettoyage selon l'une quelconque des revendications précédentes, **caractérisé en ce que** les tronçons parois de canaux (3a, 3b) sont réalisés sous la forme de tuyaux élastomères, en particulier sous la forme de tuyaux en silicone.

6. Système de nettoyage selon l'une quelconque des revendications précédentes, **caractérisé en ce que** les éléments de pinçage (5) de l'unité de mouvement (4) compriment les tronçons parois de canaux (3a, 3b) déformable radialement de l'intérieur et/ou de l'extérieur, par rapport à un centre (M) et/ou un axe médian du sélecteur de canaux.

7. Système de nettoyage selon l'une quelconque des revendications 2 à 6, **caractérisé en ce que** les éléments de pinçage (5) sont répartis uniformément sur la circonférence de l'unité de mouvement en forme de tambour.

8. Système de nettoyage selon l'une quelconque des revendications précédentes, **caractérisé en ce que** l'unité de mouvement (4) comporte différents plans (6.1, 6.2) munis d'éléments de pinçage (5), lesdits éléments de pinçage des plans respectifs étant montés de préférence en étant décalés circonférentiellement les uns par rapport aux autres.

9. Système de nettoyage selon l'une quelconque des revendications précédentes, **caractérisé en ce que** le sélecteur de canaux comporte un carter (7).

10. Système de nettoyage selon la revendication 9, **caractérisé en ce que** l'unité de mouvement (4) est logée dans le carter (7) du sélecteur de canaux.

11. Système de nettoyage selon la revendication 9, **caractérisé en ce que** l'unité de mouvement (4) est fixée sur un axe (8) monté dans le carter (7) du sélecteur de canaux.

12. Système de nettoyage selon la revendication 11, **caractérisé en ce que** l'unité de mouvement peut être entraînée en rotation par rapport au carter (7) par l'intermédiaire d'un moteur, par voie pneumatique, hydraulique et/ou manuellement.

13. Système de nettoyage selon l'une quelconque des revendications 9 à 12, **caractérisé en ce que** le carter (7) du sélecteur de canaux a des orifices d'introduction (8) pour les tuyaux flexibles élastomères, dont l'intérieur constitue les canaux (2).

14. Système de nettoyage selon l'une quelconque des revendications précédentes, **caractérisé en ce que** le nombre m satisfait à l'équation m = n - 1.
